(19) Europäisches Patentamt
European Patent Office
Office européen des brevets

(11) Veröffentlichungsnummer: **0 214 647 B1**

# (12) EUROPÄISCHE PATENTSCHRIFT

(45) Veröffentlichungstag der Patentschrift: **24.03.93**

(51) Int. Cl.5: **C08B 37/16**, A61K 31/735

(21) Anmeldenummer: **86112466.7**

(22) Anmeldetag: **09.09.86**

Die Akte enthält technische Angaben, die nach dem Eingang der Anmeldung eingereicht wurden und die nicht in dieser Patentschrift enthalten sind.

(54) **Inklusionskomplexe von 7-Isopropoxy-isoflavon mit Cyclodextrinen, Verfahren zu deren Herstellung und diese Inklusionskomplexe enthaltende pharmazeutische Präparate.**

(30) Priorität: **10.09.85 HU 341585**

(43) Veröffentlichungstag der Anmeldung:
**18.03.87 Patentblatt 87/12**

(45) Bekanntmachung des Hinweises auf die Patenterteilung:
**24.03.93 Patentblatt 93/12**

(84) Benannte Vertragsstaaten:
**AT BE CH DE FR GB IT LI NL SE**

(56) Entgegenhaltungen:
**EP-A- 0 129 893**
**FR-A- 2 530 143**

**PROCEEDINGS OF THE FIRST INTERNATIONAL SYMPOSIUM ON CYCLODEXTRINS, Budapest, 30. September - 2. Oktober 1981, Seiten 377-388, D. Reidel Publishing Co., Dordrecht, NL; A. STADLER-SZÖKE et al.: "A forecast for application of cyclodextrins in the pharma-industry"**

**Einführung in die organische Chemie, p. 366,444, (Klages, Walter de Gruyter & Co, Berlin 1961);**

(73) Patentinhaber: **CHINOIN Gyogyszer és Vegyészeti Termékek Gyára RT.**
**To utca 1-5**
**H-1045 Budapest IV(HU)**

(72) Erfinder: **Stadler, Agnes**
**N pstadion u. 18**
**H-1143 Budapest(HU)**
Erfinder: **Szejtli, J zsef**
**Endr b di S.u. 38-40**
**H-1026 Budapest(HU)**
Erfinder: **Weiszfeiler, Viktor**
**Németvölgyi u. 72/b**
**H-1124 Budapest(HU)**
Erfinder: **Vargay, Zolt n**
**Vez r u. 59/b**
**H-1144 Budapest(HU)**
Erfinder: **Kál y, Katalin**
**Ibrahim u. 15**
**H-1113 Budapest(HU)**
Erfinder: **Gergely, Vera**
**Tin di u. 15**
**H-1095 Budapest(HU)**

EP 0 214 647 B1

Erfinder: **Szüts, Tamás**
**Csalogány u. 53**
**H-1027 Budapest(HU)**

(74) Vertreter: **Bartsch, Elisabeth, Dr.**
**Patentanwälte Lotterhos & Partner Lichten-**
**steinstrasse 3**
**W-6000 Frankfurt am Main 1 (DE)**

# EP 0 214 647 B1

## Beschreibung

Die Erfindung betrifft Inklusionskomplexe von 7-Isopropoxy-isoflavon mit Cyclodextrin bzw. Cyclodextrinderivaten sowie Verfahren zur Herstellung derartiger Inklusionskomplexe. Gemäß Erfindung können als Cyclodextrin $\alpha$-, $\beta$- oder $\gamma$ -Cyclodextrin, Heptakis-2,6-O-dimethyl- bzw. Heptakis-2,3,6-tri-O-methyl-$\beta$-cyclodextrin oder ein wasserlösliches $\beta$-Cyclodextrinpolymer (MG. über 10.000) verwendet werden.

Die Erfindung betrifft weiterhin pharmazeutische Präparate, die Inklusionskomplexe von 7-Isopropoxy-isoflavon mit Cyclodextrin als Wirkstoff enthalten.

Das 7-Isopropoxy-isoflavon (Ipriflavon) wird aus den per os verabreichten Zubereitungen der Erfindung in nicht metabolisierter Form und in pharmakologisch aktiver Konzentration resorbiert und kann im Blut bestimmt werden.

Das Ipriflavon hat ein Molekulargewicht von 280,3, einen Schmelzpunkt von 112 - 118°C und eine Wasserlöslichkeit von 1 - 2 $\mu$g/ml bei 25°C. Seine Löslichkeit in Aceton und Ethanol ist ebenfalls gering, die in Chloroform und Dimethylformamid ist dagegen hoch. Seine Herstellung ist in der GB-PS 1 360 461 beschrieben.

Der Wirkstoff Ipriflavon kann als Yambolap Tablette (200 mg) zur medikamentösen Behandlung von Osteoporose und Osteomalicia verwendet werden. Die therapeutische Wirksamkeit wird jedoch durch die geringe Auslösung und Resorption beeinträchtigt. Nach einer Behandlung von Beagle-Hunden mit radioaktiv markiertem Ipriflavon ergab die Bestimmung der Radioaktivität im Urin, daß nur 12 % der zugeführten Radioaktivität mit dem Urin ausgeschieden wurde. Durch HPLC-Untersuchungen konnten die Metabolite (Hauptmetabolit ist das 7-Hydroxy-isoflavon) im Blut bestimmt werden.

Es wurden Untersuchungen durchgeführt, um die Resorption von Ipriflavon und dessen Hauptmetabolit, dem 7-Hydroxy-isoflavon, zu verbessern. Nach der europäischen Patentanmeldung 0 129 893 zeigen die schlecht lösbaren und resorbierbaren Wirkstoffe nach dem Vermahlen mit inerten Trägerstoffen eine verbesserte Lösbarkeit und Resorbierbarkeit. Werden zum Beispiel 2 g Ipriflavon mit 2 g Aerosil oder 2 g Aktivkohle oder 2 g aktiver Tonerde oder 2 g aktivem Aluminiumoxyd in einer Vibrationsmühle mit Stahlkugeln 10 - 60 Minuten vermahlen, so wird zum Beispiel mit Aerosil ein Produkt erhalten, dessen Lösbarkeit in 50 %igem wäßrigem Methanol von 37°C nach 30 Minuten 20,4 $\mu$g/ml beträgt, gegenüber einer Lösbarkeit der physikalischen Mischung ohne Vermahlen von nur 15,0 $\mu$g/ml. Dies entspricht einer 1,36-fachen Erhöhung der in vitro-Auslösung. Bei Untersuchung des Blutes von Hunden konnte nach oraler Verabreichung kein Ipriflavon festgestellt werden, durch Vermahlen mit Aerosil wurde die Resorption des Hauptmetabolits von 0,152 $\mu$g. h/ml auf 0,485 $\mu$g.h/ml erhöht, was einer 3,19-fachen Erhöhung entspricht.

Es ist bekannt, daß verschiedene leichtflüchtige und instabile pharmazeutische Wirkstoffe und Pflanzenschutzmittel durch Bildung von Molekular-Kapseln mit Cyclodextrinen in stabile kristalline Stoffe übergeführt werden können. In pharmazeutischer Hinsicht ist von großer Bedeutung, daß die Cyclodextrinkomplexe von in Wasser schlecht lösbaren Wirkstoffen, in Wasser leicht benetzt, rasch resorbiert und somit rasch gelöst werden können. Die Grenze der Lösbarkeit und Resorption wird bedeutend erhöht, wobei diese Erhöhung im Verhältnis zum nicht komplexierten Wirkstoff einen Wert von 1,3 - 3 erreicht (W.F.Smolen und L.A.Ball: Controlled drug bioavailability, Vol. 3, John Wiley, New York, 1985 p. 365).

Es wurde gefunden, daß die in vitro-Lösbarkeit von Ipriflavon durch die Bildung von Inklusionskomplexen mit Cyclodextrin oder Cyclodextrinderivaten auf das 5-fache und die Blutspiegel des Hauptmetaboliten auf das 15 - 20-fache erhöht werden kann. Dadurch wird gleichzeitig die Resorption von Ipriflavon in unveränderter Form in pharmazeutisch aktiver Konzentration ermöglicht.

Die Cyclodextrine werden aus Stärke mit Hilfe des Cyclodextringlycosyl-Transferase-Enzyms hergestellt. Es lassen sich drei verschiedene Cyclodextrine unterscheiden: das $\alpha$-, das $\beta$- und das $\gamma$-Cyclodextrin, die aus 6, 7 oder 8 Glycopyranose-Einheiten aufgebaut sind, die miteinander durch $\alpha$-1,4-Glycosidbindungen verbunden sind. Diese drei Arten von Cyclodextrinen unterscheiden sich im Molekulargewicht, in der Wasserlöslichkeit und im Durchmesser des Hohlraums voneinander. Infolgedessen können diese Inklusionskomplexe die verschiedensten Verbindung bilden, wobei die Inklusionskomplexe von einer Verbindung mit verschiedenen Cyclodextrinen große Unterschiede im Verhalten zeigen.

Durch Substitution können die Cyclodextrine modifiziert werden. So sind zum Beispiel im Dimethyl-$\beta$-cyclodextrin (DIMEB) zwei Hydroxylgruppen der Glucose-Einheit methyliert, und im Trimethylcyclodextrin (TRIMEB) sind alle Hydroxylgruppen durch Methoxy ersetzt. Diese substituierten Cyclodextrine weisen eine bessere Lösbarkeit und unterschiedliche komplexbildende Wirkung - verglichen mit den unsubstituierten Cyclodextrinen - auf. Allgemeine Charakteristica der Cyclodextrine sind die hydrophile Außenoberfläche und die apolare Innenoberfläche der Moleküle, welche eine entsprechende Größe besitzen muß, um in sich Gastmoleküle zu binden und dadurch eine sogenannte Moleküleinkapselung zu bewirken.

3

Ein Beweis für die Bildung von Inklusionskomplexen des Ipriflavons mit Cyclodextrinen ist die Erhöhung der Lösbarkeit des Ipriflavons in wäßrigen Lösungen. Zu diesem Zweck wurden in 10 ml Prüfgläser 10 mg Ipriflavon und 10 mg der verschiedenen Cyclodextrine gegeben, danach 5 ml destilliertes Wasser zugesetzt und die verschlossenen Gläser 4 Tage bei 25°C mit 325 rpm geschüttelt. Danach wurden die Proben über eine G4 Glasfritte filtriert und der Ansatz in 50 %igem wäßrigen Methanol photometriert. Die Ergebnisse sind in Tabelle I zusammengestellt.

Tabelle I

Lösbarkeit von Ipriflavon (µg/ml) in wäßrigen Cyclodextrinlösungen verschiedener Konzentrationen bei 25°C

| Cyclo-dextrin mg/ml | α-CD | β-CD | γ-CD | DIMEB | TRIMEB |
|---|---|---|---|---|---|
| 1 | | 2,52 | | | |
| 3 | | 7,03 | | 22,0 | |
| 5 | | 11,44 | | 40,6 | |
| 7 | | 15,93 | | 59,1 | |
| 9 | | 19,51 | | 77,5 | |
| 11 | 4,12 | 15,22 | 2,02 | 96,2 | 8,23 |
| 13 | 4,68 | 15,48 | 2,05 | 114,8 | 9,88 |
| 15 | 5,08 | 15,75 | 2,16 | 191,3 | 11,32 |
| 20 | 6,10 | 13,46 | 2,38 | 284,4 | 14,71 |
| 30 | 8,19 | 7,59 | 2,74 | 416,8 | 26,82 |
| 40 | 9,63 | 6,20 | 3,26 | 588,2 | 43,91 |
| 50 | 10,61 | 5,44 | 3,04 | 761,0 | 61,3 |
| 70 | 14,77 | 6,08 | 3,64 | 1118 | 96,2 |
| 90 | 18,24 | 6,93 | 4,51 | 1142 | 127,5 |
| 100 | 21,63 | 6,64 | 4,76 | 1654 | 179,0 |
| 110 | 19,02 | 5,98 | 5,12 | 1824 | 218,8 |
| 120 | 23,65 | 5,66 | 5,48 | 2012 | 269,3 |
| 150 | | | | 2024 | 435,6 |

Die Lösbarkeit des Ipriflavons beträgt in destilliertem Wasser bei 25°C 1 - 2 µg/ml. Tabelle I ist zu entnehmen, daß sich die Lösbarkeit von Ipriflavon in β-Cyclodextrinlösungen in Abhängigkeit von der Konzentration des Cyclodextrins bis auf das 15-fache des in destilliertem Wasser gemessenen Wertes erhöhen kann. Bei einer Konzentration des β-Cyclodextrins von etwa 1 % erhöht sich die Lösbarkeit des Ipriflavons bis auf etwa 16 - 20 µg/ml, bei weiterer Erhöhung der Cyclodextrinkonzentration bleibt die Lösbarkeit des Ipriflavons unverändert, nach Erreichen einer Cyclodextrinkonzentration von 1,8 % nimmt die Lösbarkeit ab und bei Cyclodextrinkonzentrationen oberhalb von 4 % stellt sich ein Standardwert von etwa 6 µg/ml ein. Dieser Wert kann als die Lösbarkeit des festen Komplexes - gebildet in überschüssigem Ipriflavon - angesehen werden.

Die Lösbarkeit von Ipriflavon kann in Lösungen von γ-Cyclodextrinen nicht bedeutend erhöht werden. Deren Wert entspricht etwa einer 3-fachen Erhöhung in 15 %igen Cyclodextrinlösungen.

Bei α-Cyclodextrin kann bei einer Konzentration von 12 % eine Lösbarkeit von 20 - 22 µg/ml erreicht werden, wobei jedoch ein verändertes UV-Spektrum von Ipriflavon zu beobachten ist.

Durch Verwendung von TRIMEB bzw. DIMEB kann eine größere Erhöhung der Lösbarkeit erreicht werden. Bei Erhöhung der Cyclodextrinkonzentration ist eine nahezu lineare Erhöhung der Lösbarkeit zu beobachten. Deren Wert beträgt in 15 %iger TRIMEB-Lösung 440 $\mu$g/ml, in 15 %iger DIMEB-Lösung 1700 - 2000 $\mu$g/ml, was einer 300-fachen bzw. 1700-fachen Erhöhung entspricht.

Zusammenfassend ist festzustellen, daß bei den Ipriflavon-Inklusionskomplexen der Erfindung die Wasserlöslichkeit mindestens um das 4-fache und die Resorption bei Ratten nach oraler Verabreichung mindestens um das 10-fache gegenüber dem freien Wirkstoff erhöht wurde, und daß der Anteil an unverändertem 7-Isopropoxy-isoflavon mindestens 25 % der Gesamtmenge des resorbierten Stoffes beträgt.

Die Inklusionskomplexe der Erfindung werden durch Umsetzung von 7-Isopropoxy-isoflavon mit Cyclodextrin bzw. Cyclodextrinderivat in wäßrig-organischem Medium hergestellt.

Als organische Lösungsmittel werden vorzugsweise Alkohole und Ketone verwendet.

Bei der Durchführung der Umsetzung wird vorzugsweise so vorgegangen, daß aus den Komponenten in wäßriger Ethanollösung (bevorzugt wird eine Ethanolkonzentration von etwa 50 %) eine homogene Lösung gebildet und aus dieser der kristalline Inklusionskomplexe durch Kühlen ausgefällt wird.

Die alternative Möglichkeit besteht in dem mechanischen Zusammenkneten der Komponenten in wäßrig-acetonischem Medium bei gleichzeitiger Entfernung des Lösungsmittels. Bei Verwendung von DIMEB, TRIMEB oder lösbaren Cyclodextrinpolymeren kann das Endprodukt aus der Lösung durch Eindampfen, Sprühtrocknung oder Gefriertrocknung, bzw. bei Verwendung von DIMEB durch Erwärmen, in Form von festem Pulver gewonnen werden.

Die Erfindung wird durch folgende Beispiele näher erläutert, ohne das Schutzbegehren auf diese Beispiele einzuschränken.

Beispiele zur Herstellung von Ipriflavon-Cyclodextrin-Komplexen

Beispiel 1

Ipriflavon-$\beta$-Cyclodextrin-Komplex mit einem Molverhältnis von 1 : 2 Man löst 11,0 g (39,2 mMol) Ipriflavon und 114,3 g (88,3 mMol) $\beta$-Cyclodextrin mit einem Wassergehalt von 12,36 % in 2,25 Liter einer 50 %igen Ethanollösung in destilliertem Wasser bei 80°C, rührt die Lösung intensiv und läßt sie auf Zimmertemperatur abkühlen. Das ausgefallene kristalline Produkt wird abfiltriert und 1 Tag bei 60°C getrocknet. Die Ausbeute beträgt 96,4 g, der Ipriflavongehalt 10,8 Gew.% (Ipriflavon : $\beta$-Cyclodextrin-Molverhältnis = 1 : 2,0) und die Ausbeute auf den Wirkstoff bezogen: 94,7 %. Das erhaltene Produkt ist ein lockeres weißes Pulver mit einer Korngröße unter 90 $\mu$m. Die Tatsache, daß es sich bei dem erhaltenen Produkt um einen Inklusionskomplex handelt, wurde durch thermoanalytische Untersuchungen, Röntgendiffraktions- und Lösungsuntersuchungen bewiesen.

Das thermoanalytische Verhalten einer mechanischen Mischung, deren Zusammensetzung der des Inklusionskomplexes entspricht, ist ähnlich dem des freien Wirkstoffes, solange die beobachtete Veränderung der Komplexe auf Wärme hin nur auf dem gleichzeitigen Zerfall des Cyclodextrins beruht. Bei der Differential Scanning Calorimetrie (DSC) von mechanischen Mischungen entsteht eine endothermische Spitze bei 115°C, wie beim Schmelzen des freien Ipriflavons. Diese Spitze tritt bei Inklusionskomplexen nicht auf. Die mechanischen Mischungen zeigen zwischen 130 - 250°C eine ständige Gewichtsabnahme, die der Menge des freien Ipriflavons entspricht. Die Inklusionskomplexe weisen dagegen in diesem Bereich keine Gewichtsveränderungen auf (Thermal Evolution Analysis, TEA).

Das Röntgendiffraktionspulverdiagramm der mechanischen Mischungen setzt sich aus den Diagrammen der Komponenten additiv zusammen. Das Diagramm des Komplexes weist nur wenige Spitzen auf, und diese Spitzen (2 $\theta$° = 6,7; 7,8; 20,9), weichen von den Spitzen des $\beta$-Cyclodextrins (2 $\theta$° = 4,5; 10,6; 12,3; 16,1; 19,5) und denen des Ipriflavons (2 $\theta$° = 5,8; 11,5; 15,5; 17,3; 22,0) ab. Dies bedeutet, daß sich eine neue Kristallstruktur, d.h. ein Inklusionskomplex, gebildet hat.

Um die Lösbarkeit zu prüfen, wurden in 500 ml Kolben 250 ml destilliertes Wasser gegeben und thermostatisch auf 37°C gehalten, danach Proben, die 50 mg Wirkstoff entsprechen, zugefügt und die gebildete Suspension mit einem magnetischen Rührer mit einer Geschwindigkeit von 1000 rpm gerührt. Es wurden Proben mit einem Volumen von 2 - 6 ml herausgenommen und über eine G4 Glasfritte filtriert. Das Filtrat wurde nach dem Verdünnen in 1 : 1 wäßrigem Ethanol photometriert. Die Ergebnisse sind in Tabelle II zusammengestellt.

Tabelle II

Lösbarkeit des Ipriflavons, des Ipriflavon-ß-Cyclodextrin-Komplexes

des Beispiels 1 und einer Mischung von Ipriflavon und ß-Cyclodextrin

in 250 ml destilliertem Wasser bei 37°C

| Zeit (Min) | Konzentration des gelösten Ipriflavons (µg/ml) | | |
| --- | --- | --- | --- |
| | Ipriflavon 51,5 mg | Ipriflavon-ß-Cyclodextrin-Komplex 471,5 mg | Mischung von Ipriflavon und ß-Cyclodextrin 444,7 mg |
| 1 | 0,435 | 13,21 | 0,442 |
| 2 | 0,327 | 9,26 | 0,462 |
| 3 | 0,401 | 8,03 | 0,726 |
| 5 | 0,507 | 7,36 | 1,20 |
| 10 | 0,882 | 7,62 | 2,28 |
| 15 | 1,123 | 7,43 | 3,21 |
| 20 | 1,197 | 7,89 | 4,69 |
| 30 | 1,423 | 6,89 | 5,86 |
| 40 | 1,508 | 7,11 | 6,07 |
| 60 | 1,621 | 6,81 | 6,27 |
| 90 | 1,525 | 6,89 | 6,34 |
| 120 | 1,482 | 6,94 | 6,37 |

Die Lösbarkeit des freien Wirkstoffes ist ziemlich gering, nach 40 - 50 Minuten wird die Ipriflavon-Sättigung erreicht, die einer Menge von 1,5 µg/ml entspricht. Bei Ipriflavon-$\beta$-Cyclodextrin-Komplexen konnte nach 1 Minute eine maximale Ipriflavonkonzentration gemessen werden, was ein Anzeichen für eine außerordentlich schnelle Auflösung ist, wobei am Anfang ein doppelter Sättigungswert (7 µg/ml) beobachtet wird. Die Lösbarkeit von Mischungen des Ipriflavons und des $\beta$-Cyclodextrins ist langsam und erreicht die bei den Komplexen gemessene Ipriflavonkonzentration erst nach 30 - 40 Minuten. Untersuchungen des Einflusses des pH-Wertes auf die Lösbarkeit ergaben, daß die beim pH des Magens (pH 1,3, Salzsäure) und beim pH des Darms (pH 7,6, Phosphatpuffer) erhaltenen Untersuchungsergebnisse von den obigen Ergebnissen nicht wesentlich abweichen, d.h. die Lösbarkeit ist von dem pH-Wert in dem untersuchten Bereich unabhängig.

Beispiel 2

Ipriflavon-$\gamma$-Cyclodextrin-Komplex mit einem Molverhältnis von 1 : 2 Man löst 8,0 g (5,35 mMol) $\gamma$-Cyclodextrin mit einem Wassergehalt von 13,3 % in 50 ml destilliertem Wasser bei 60°C, tropft dieser Lösung innerhalb von 2 Stunden unter intensivem Rühren eine Lösung von 0,5 g (1,78 mMol) Ipriflavon in 50 ml 96 %igem Ethanol zu, und läßt die erhaltene Lösung auf Zimmertemperatur abkühlen. Man rührt die Lösung intensiv weitere 16 Stunden, filtriert das ausgefallene Produkt ab und trocknet es 1 Tag bei 60°C. Ausbeute: 5,5 g, Ipriflavongehalt:7,9 Gew.% (Ipriflavon : $\gamma$-Cyclodextrin-Molverhältnis = 1 : 2,29). Ausbeute auf den Wirkstoff bezogen: 87 %. Das Produkt enthält freies $\gamma$-Cyclodextrin.

Beispiel 3

Ipriflavon-$\alpha$-Cyclodextrin-Komplex mit einem Molverhältnis von 1 : 2 Man löst 0,5 g (1,78 mMol) Ipriflavon in 10 ml Aceton im Mörser und fügt 4,0 g (3,70 mMol) $\alpha$-Cyclodextrin mit einem Wassergehalt von 10 %, sowie 2 ml destilliertes Wasser zu. Die erhaltene dünne Suspension wird unter ständigem Rühren bis zum Abdampfen des Lösungsmittels homogenisiert. Das so erhaltene Produkt wird 1 Tag bei 60°C getrocknet. Das Produkt enthält 11,1 % Wirkstoff (Ipriflavon : α-Cyclodextrin-Molverhältnis = 1 :2,1) und daneben freies α-Cyclodextrin.

Beispiele für die Herstellung von pharmazeutischen Zubereitungen, die den Ipriflavon-β-Cyclodextrin-Komplex enthalten, und für die biologische Resorption des Wirkstoffes.

Beispiel 4

Das Auslösen der Wirkstoffe aus einer handelsüblichen Yambolap Tablette mit 200 mg Ipriflavon (Tablette A), sowie aus Tabletten mit 40 mg Wirkstoff in Form des β-Cyclodextrin-Inklusionskomplexes des Beispiels 1 (Tablette B), bzw. aus einer Mischung von 200 mg Ipriflavon und 330 mg β-Cyclodextrin (Tablette C) wurde mit einem Erweka ZT-4 Apparat bei 37°C in 500 ml Magenflüssigkeit bestimmt.

Zu der Bestimmung wurde 1 Tablette in den Apparat eingelegt und die Menge des herauslösbaren Stoffes spektrophotometrisch (247 nm) gemessen. Die untersuchten Tabletten haben folgende Zusammensetzung:

Tablette A (handelsübliche Yambolap (K-020484)):

| | |
|---|---|
| Ipriflavon | 200 mg |
| Amylum maydis | 36 mg |
| Lactosum | 60 mg |
| PVP | 13 mg |
| Esma Spreng | 30 mg |
| Talcum | 7 mg |
| Magnesium stearinicum | <u>4 mg</u> |
| | 350 mg |

| | |
|---|---|
| Tablette B Komplex (enthält 40 mg Ipriflavon) | 370 mg |
| PVP | <u>15 mg</u> |
| | 385 mg |

| | |
|---|---|
| Tablette C Ipriflavon | 200 mg |
| ß-Cyclodextrin | 330 mg |
| PVP | <u>20 mg</u> |
| | 550 mg |

Die Ergebnisse der Auslöseversuche sind in Tabelle III zusammengestellt.

Tabelle III

Menge des aus den verschiedenen Tabletten herausgelösten Wirkstoffes

| Zeit (Min) | herausgelöster Wirkstoff (mg) | | |
|---|---|---|---|
| | A | B | C |
| 2 | – | 0,80 | – |
| 5 | 0,21 | 1,05 | 0,27 |
| 10 | 0,45 | 1,10 | 0,50 |
| 15 | 0,58 | 1,15 | 0,60 |
| 30 | 0,56 | 1,15 | 0,65 |
| 60 | 0,61 | 1,30 | 0,75 |
| 120 | 0,60 | 1,30 | 1,00 |
| 180 | 0,62 | 1,40 | 1,15 |

Aus der handelsüblichen Tablette A wurden nur 0,62 mg Ipriflavon herausgelöst. Aus Tablette B, die in Form des $\beta$-Cyclodextrinkomplexes nur 1/5 des Wirkstoffes enthält, wurde mehr als die 2-fache Menge des Wirkstoffes herausgelöst, wobei diese Menge nach 2 Minuten um 30 % größer war, als die maximale Menge bei der handelsüblichen Tablette A. Bei Tablette C, die eine mechanische Mischung von Ipriflavon und $\beta$-Cyclodextrin enthält, war die Auslösegeschwindigkeit zwar geringer, die Menge des herausgelösten Wirkstoffes aber größer als die der handelsüblichen Tablette A.

Die Resorptionsversuche wurden mit männlichen Ratten vom Typ CFY durchgeführt. Das Gewicht der Testtiere lag zwischen 170 und 200 g. Vor der Behandlung ließ man die Testtiere hungern, nach der Behandlung gab man ihnen Wasser und Rattenfutter ad libitum. Das Ipriflavon wurde in einer Dosis von 25 mg/kg, und der Ipriflavon-$\beta$-Cyclodextrinkomplex des Beispiels 1 wurde in einer äquimolaren Dosis - auf den Wirkstoff bezogen - verabreicht. Die mit Ipriflavon behandelten Testtiere wurden als Gruppe A, die mit der mechanischen Mischung behandelten Tiere als Gruppe C und die mit dem Irpiflavon-$\beta$-Cyclodextrinkomplex behandelten Tiere als Gruppe B bezeichnet.

Die abgemessene Menge an den Stoffen wurde 5 Minuten in 2 ml einer 1 %igen Methylcelluloselösung gerührt und den Tieren über eine Magensonde zugeführt. Nach 10 und 30 Minuten bzw. 1, 2 und 4 Stunden ließ man die Testtiere nach Durchschneiden der Vena femoralis in Ethernarkose ausbluten, sammelte das Blut und zentrifugierte es in heparinisierten Röhren. Das Plasma wurde abgetrennt und bis zum Aufarbeiten in Ampullen tiefgekühlt.

Um den Urin zu sammeln, wurden die Testtiere in Einzel-metabolismusgefäße gelegt und der Urin und die Exkremente 24 Stunden getrennt gesammelt. Die Proben wurden bis zum Aufarbeiten in Ampullen tiefgekühlt.

Die Konzentration des Ipriflavons und dessen Hauptmetabolits (7-Hydroxy-isoflavon) wurde im Blut und im Urin durch HPLC bestimmt.

Die Bestimmung wurde im HP 1082B Flüssigkeitschromatograph in einer 20 cm RP-18 LiChrosorb (Korngröße bei Plasmaproben 10 $\mu$m, bei Urinproben 5 $\mu$m) Drehphasenkolonne durchgeführt. Die Fließgeschwindigkeit des Eluents betrug 1,5 ml/Minute. Der UV-Detektor wurde auf 254 nm eingestellt und die Chromatogramme bei einer Papiergeschwindigkeit von 0,2 cm/Minute aufgenommen. Als Eluent wurde eine Mischung von 0,05 Mol Acetatpuffer (pH 3) und Acetonitril (Interkémia) 60 : 40 bei Plasmaproben und von 55 : 45 bei Urinproben verwendet. Als Innerstandard wurde eine Lösung von 2-Methyl-7-methoxy-4'-nitro-isoflavon in Ethylalkohol der Konzentration von 100 $\mu$g/ml, und zur Kalibration wurden die Lösungen von 7-Hydroxy-isoflavon in Methylalkohol der Konzentrationen 100 $\mu$g/ml und 10 $\mu$g/ml verwendet.

Zur Analyse der Plasmaprobe wurde 1 ml Plasma mit 1 ml pH 5 Acetatpuffer und 10 $\mu$l $\beta$-Glucuronidase/Arylsulfatase-Enzym (100 FU), mit destilliertem Wasser auf das 10-fache verdünnt, vereinigt und 24 Stunden bei 37° C inkubiert. Danach wurde 50 $\mu$l 2 n NaOH zugefügt, mit 8 ml Benzol extrahiert und zentrifugiert. Die organische Phase wurde abgetrennt und die wäßrige Phase mit 400 $\mu$l 1 n HCl angesäuert, mit 10 $\mu$l Innerstandard vermischt und mit 8 ml Benzol erneut extrahiert. Nach dem Zentrifugie-

8

ren wurde die Benzolphase eingedampft, der Rückstand in 100 $\mu$l Eluent gelöst und in einer Menge von 50 $\mu$l injektiert. Die Gleichung der Plasmakalibrationslinie ist:

y = 8,2x - 0,08, der Korrelationskoeffizient beträgt 0,9999.

Zur Analyse der Urinprobe wurden 0,5 ml Urin mit 0,5 ml pH 5 Acetatpuffer und 10 $\mu$l $\beta$-Glucuronidase/Arylsulfatase-Enzym (1000 FU) vereinigt und 24 Stunden bei 37°C inkubiert. Danach gab man 150 $\mu$l 2 n NaOH zu, extrahierte mit 8 ml Benzol und zentrifugierte. Die organische Phase wurde abgetrennt und die wäßrige Phase mit 400 $\mu$l 1 n HCl angesäuert, mit 50 $\mu$l Innerstandard vermischt und mit 8 ml Benzol erneut extrahiert. Nach dem Zentrifugieren wurde die Benzolphase eingedampft, der Rückstand in 100 $\mu$l Eluent aufgenommen und in einer Menge von 10 $\mu$l in den Flüssigkeitschromatographen injektiert. Die Gleichung der Urinkalibrationslinie ist: y = 0,911x - 0,09, der Korrelationskoeffizient beträft 0,9995.

Die Tabelle IV zeigt die Konzentration des unveränderten Irpiflavons und die Tabelle V die des Hauptmetaboliten im Blut der Testtiere. Die Gruppe A wurde mit freiem Ipriflavon, die Gruppe B mit dem Komplex und die Gruppe C mit der Mischung von Irpiflavon und $\beta$-Cyclodextrin behandelt.

Tabelle IV

Plasmakonzentration des unveränderten Ipriflavons in Abhängigkeit von der Zeit nach der p.o. Verabreichung äquivalenter Wirkstoffdosen

| Zeit (h) | A Ipriflavon | B Ipriflavon-ß-Cyclodextrin-Komplex des Beispiels 1 | C Mischung von Ipriflavon und ß-Cyclodextrin |
|---|---|---|---|
| 0,17 | 0 | 0,065 | 0,479 |
| | 0 | 0,055 | 0 |
| | 0 | 0,073 | 0 |
| 0,5 | 0 | 0,468 | 0 |
| | 0 | 0,149 | 0 |
| | 0 | 0,194 | 0 |
| 1 | 0 | 0,271 | 0 |
| | 0 | 0,354 | 0 |
| | 0 | 0,293 | 0 |
| 2 | 0,078 | 0,514 | 0,081 |
| | 0 | 0,402 | 0 |
| | 0,095 | 0,214 | 0 |
| 4 | 0,041 | 0,148 | 0 |
| | 0 | 0,363 | 0 |
| | 0 | 0,054 | 0 |
| 24 | 0 | 0 | 0 |
| | 0 | 0 | 0 |
| | 0 | 0 | 0 |

## Tabelle V

Plasmakonzentration des Hauptmetaboliten (7-Hydroxy-isoflavon) in Abhängigkeit von der Zeit nach der p.o. Verabreichung von äquivalenten Wirkstoffdosen

| Zeit (h) | A | B Ipriflavon-ß-Cyclodextrin-Komplex des Beispiels 1 | C Mischung von Irpiflavon und ß-Cyclodextrin |
|---|---|---|---|
| 0,17 | 0,156 | – | 2,080 |
| | 0,265 | 2,450 | 0,457 |
| | 0,195 | 3,650 | 0,767 |
| 0,5 | 0,860 | 12,270 | 0,304 |
| | 0,591 | 4,840 | 0,374 |
| | 0,365 | – | 0,358 |
| 1 | 0,239 | 12,190 | 0,306 |
| | 0,790 | 4,330 | – |
| | 0,340 | 3,870 | 0,274 |
| 2 | 1,218 | 22,040 | 0,270 |
| | 0,715 | – | 0,358 |
| | 1,093 | 3,780 | 0,826 |
| 4 | 0,220 | 1,740 | – |
| | 0,237 | – | 0,130 |
| | 0,235 | 2,520 | 0,330 |
| 24 | 0 | 0 | 0 |
| | 0 | 0 | 0 |
| | 0 | 0 | 0 |

Unverändertes Ipriflavon konnte im Plasma zu allen Zeitpunkten nur bei Gruppe B bestimmt werden. Der Wert liegt zwischen 50 und 500 ng/ml. Bei den Gruppen A und C konnte unverändertes Ipriflavon nur zu bestimmten Zeitpunkten (2 und 4 Stunden) gemessen werden. Bezüglich des Hauptmetaboliten konnte bei der Gruppe B zu allen Zeitpunkten eine 10 - 20-fache Plasmakonzentration gegenüber der der Gruppe A und C gemessen werden. Die Werte liegen bei der Gruppe B zwischen 3 und 20 $\mu$g/ml, bei den Gruppen A und C zwischen 0,2 und 1 $\mu$g/ml. In allen Fällen konnte nach 2 Stunden ein zweites Maximum bei der Plasmakurve beobachtet werden, was die Folge eines intensiven enterohepatischen Kreislaufs und der besseren Resorption aus dem Dünndarm sein kann.

Die Tabelle VI zeigt die Ergebnisse der HPLC Bestimmung des Urins. Unverändertes Ipriflavon konnte in den Urinproben der Zeitpunkte 0 bis 24 Stunden nicht gemessen werden. Der Hauptmetabolit konnte in allen Fällen nachgewiesen werden. Der Durchschnittswert beträgt bei Gruppe B 952 $\mu$g 7-Hydroxy-isoflavon, dies bedeutet etwa der 3-fache Wert gegenüber denen der Gruppen A und C. Nach der gewonnenen Erfahrung macht die Menge des Hauptmetaboliten 50 - 55 % der Gesamtmetaboliten aus, das heißt, die Gesamtmenge der durch den Urin ausgeschiedenen Stoffe ist etwa doppelt so groß wie die gemessenen Werte. So wurde bei Gruppe B etwa 40 %, bei den Gruppen A und C dagegen nur 10 - 14 % der verabreichten Wirkstoffdosis ausgeschieden.

Zusammenfassend kann festgestellt werden, daß bei Verabreichung des Irpiflavon-$\beta$-Cyclodextrin-Komplexes an Ratten eine 10 - 15-fache Plasmakonzentration und eine etwa 3-fache Menge, durch Urin ausgeschieden, zur Kontrolle erreichbar ist.

Tabelle VI

Ausscheiden des Hauptmetaboliten durch den Urin nach p.o.

Verabreichung äquivalenter Wirkstoffdosen

|  | Tier Nr. | Gesamtmenge der ausgeschiedenen Stoffe µg | Menge der ausgeschiedenen Stoffe m% der verabreichten Dosen (D %) | D% |
|---|---|---|---|---|
| A Ipriflavon | 1 | 236,1 | 9,44 | |
| | 2 | 344,4 | 13,77 | 11,34 |
| | 3 | 270,4 | 10,81 | |
| B Komplex gemäß Beispiel 1 | 4 | 925,4 | 37,01 | 38,09 |
| | 5 | 978,9 | 39,16 | |
| C Mischung von Ipriflavon und $\beta$-Cyclodextrin | 6 | 341,6 | 13,66 | |
| | 7 | 494,3 | 19,77 | 13,33 |
| | 8 | 163,9 | 6,56 | |

**Patentansprüche**

1. Inklusionskomplexe von 7-Isopropoxy-isoflavon mit $\alpha$-Cyclodextrin, $\beta$-Cyclodextrin, $\gamma$-Cyclodextrin, Heptakis-2,6-O-dimethyl-cyclodextrin, Heptakis-2,3-6-tri-O-methyl-$\beta$-cyclodextrin oder mit wasserlöslichem $\beta$-Cyclodextrinpolymer (Molekulargewicht über 10.000)

2. Verfahren zur Herstellung von Inklusionskomplexen von 7-Isopropoxy-isoflavon mit Cyclodextrinen des Anspruchs 1, dadurch gekennzeichnet, daß man 7-Isopropoxy-isoflavon in wäßrigorganischen Lösungsmitteln mit Cyclodextrin umsetzt.

3. Pharmazeutische Präparate, die als Wirkstoff einen 7-Isopropoxy-isoflavon-Cyclodextrin-Inklusionskomplex des Anspruchs 1 enthalten.

**Claims**

1. Inclusion complexes of 7-isopropoxy-isoflavone with $\alpha$-cyclodextrin, $\beta$-cyclodextrin, $\gamma$-cyclodextrin, heptakis-2,6-O-dimethyl-cyclodextrin, heptakis-2,3,6-tri-O-methyl-$\beta$-cyclodextrin or with a water-soluble $\beta$-cyclodextrin polymer (molecular weight: above 10 000).

2. A process for the preparation of inclusion complexes of cyclodextrins of claim 1, characterized in that 7-isopropoxy-isoflavone is reacted with cyclodextrin in aqueous/organic-solvents.

EP 0 214 647 B1

3. Pharmaceutical products that contain an 7-isopropoxy-isoflavone-cyclodextrin-inclusion complex of claim 1 as the active agent.

**Revendications**

1. Complexes d'inclusion de 7-isopropoxy-isoflavone avec l'α-cyclodextrine, la β-cyclodextrine, la γ-cyclodextrine, la heptakis-2,6-O-diméthyl-cyclodextrine, la heptakis-2,3,6-tri-O-méthyl-β-cyclodextrine ou avec un polymère de β-cyclodextrine soluble dans l'eau (poids moléculaire: plus de 10 000).

2. Procédé de préparation de complexes d'inclusion de 7-isopropoxy-isoflavone avec des cyclodextrines de la revendication 1, caractérisé en ce qu'on fait réagir le 7-isopropoxy-isoflavone avec la cyclodextrine en solvants aqueux-organiques.

3. Préparations pharmaceutiques qui contiennent comme substance active un complexe d'inclusion de la revendication 1 de 7-isopropoxy-isoflavone avec la cyclodextrine.

12